# FASCICULE DE BREVET EUROPEEN

(11) **EP 2 349 188 B1**
(45) Date de publication et mention de la délivrance du brevet: **08.04.2015**
(21) Numéro de dépôt: 09760220.5
(22) Date de dépôt: 27.10.2009
(51) Int. Cl.: A61K 8/19, A61K 8/63, A61K 8/90, A61K 31/575, A61K 31/765, A61K 9/00, A61P 17/00, A61Q 19/06

(54) **SOLUTIONS SALINES AQUEUSES POUR LA DESTRUCTION DE TISSUS GRAISSEUX**
WÄSSRIGE SOLEN ZUR ZERSTÖRUNG VON FETTGEWEBE
AQUEOUS SALINE SOLUTIONS FOR THE DESTRUCTION OF FATTY TISSUE

(30) Priorité: 27.10.2008 FR 0805960
(43) Date de publication de la demande: 03.08.2011
(73) Titulaire: Lipolyane, 69160 Tassin La Demi Lune (FR)
(72) Inventeur: CRASSAS, Yves, F-69006 Lyon (FR); BLACHE, Denis, F-21380 Messigny et Vantoux (FR)
(74) Mandataire: Verriest, Philippe
(86) Numéro de dépôt international: PCT/FR2009/052063
(87) Numéro de publication internationale: WO 2010/049637

(56) Documents cités:
- EP-A- 1 970 051
- EP-A1- 0 444 240
- EP-A2- 1 004 880
- WO-A-2005/063169
- FR-A- 2 876 907
- US-A1- 2006 127 468
- US-A1- 2006 275 841
- US-A1- 2008 176 331
- ROTUNDA ET AL: "Lipomas treated with subcutaneous deoxycholate injections" JOURNAL OF THE AMERICAN ACADEMY OF DERMATOLOGY, C.V. MOSBY, ST. LOUIS, MO, US, vol. 53, no. 6, 1 décembre 2005 (2005-12-01), pages 973-978, XP005168221 ISSN: 0190-9622
- ANGELA Y SONG ET AL: "Scientific Basis for the Use of Hypotonic Solutions with Ultrasonic Liposuction" AESTHETIC PLASTIC SURGERY, SPRINGER-VERLAG, NE, vol. 30, no. 2, 1 avril 2006 (2006-04-01), pages 233-238, XP019364489 ISSN: 1432-5241
- MANN M W ET AL: "New Advances in Liposuction Technology" SEMINARS IN CUTANEOUS MEDICINE AND SURGERY, W.B. SAUNDERS, PHILADELPHIA, US, vol. 27, no. 1, 1 mars 2008 (2008-03-01), pages 72-82, XP022646964 ISSN: 1085-5629 [extrait le 2008-05-24]
- ROTUNDA A M ET AL: "Detergent effects of sodium deoxycholate are a major feature of an injectable phosphatidylcholine formulation used for localized fat dissolution" DERMATOLOGIC SURGERY, ELSEVIER SCIENCE, NEW YORK, NY, US, vol. 30, no. 7, 1 juillet 2004 (2004-07-01), pages 1001-1008, XP002331643 ISSN: 1076-0512

## Description

La présente invention concerne des solutions salines aqueuses utilisables pour la destruction de tissus graisseux, en particulier des solutions hypotoniques. Malgré des attentions diététiques particulières et/ou une pratique sportive régulière, une accumulation graisseuse inesthétique (couramment dénommée cellulite) peut être constatée chez certaines personnes. Cette tendance est la conséquence des modifications du style de vie, conduisant aussi à une augmentation générale du surpoids, voire de l'obésité par accumulation localisée de graisses de réserve.

A cet égard, dans le cadre de la présente invention, le terme « cellulite » désigne, conformément au langage courant, une cellulite esthétique, c'est-à-dire une hypertrophie du tissu adipeux généralement très localisé formant des capitons appelés « peau d'orange ».

La cellulite est en effet une hyperlipodystrophie superficielle, alors que la stéatomérie correspond à une hyperlipodystrophie localisée et plus profonde.

Il est à noter que d'un point de vue anatomique, il existe sous la peau (qui se compose de l'épiderme et du derme) trois couches de graisse :
- la couche la plus superficielle, située dans l'hypoderme, est organisée en chambres graisseuses séparées entre elles par des cloisons conjonctives élastiques. Cette couche de « graisse hypodermique », dont le métabolisme est principalement sous contrôle hormonal, est responsable de l'apparition de la cellulite ; puis
- sous l'hypoderme, deux couches de graisse de réserve, dont le métabolisme est essentiellement sous contrôle de la génétique et du style de vie (alimentation, activité physique), à savoir :
   ∘ la « graisse parallèle » qui présente une épaisseur variable selon la corpulence du patient. Cette graisse est particulièrement sensible au régime,
   ∘ une graisse encore plus profonde, appelée « graisse stéatomérique » ou « graisse de structure ». Sa répartition sur l'organisme est irrégulière et varie selon les individus. Cette graisse est très résistante au régime et est responsable des irrégularités de la silhouette, comme la culotte de cheval.

Une des techniques actuelles pour traiter l'accumulation importante de tissus adipeux est la lipo-aspiration, acte chirurgical très pratiqué, mais qui n'est pas dénué de risques, en particulier vitaux pour les patients obèses. La lipo-aspiration nécessite en effet une longue anesthésie et laisse un grand nombre de cicatrices préjudiciables sur le plan esthétique. Il est donc aisé de comprendre l'engouement pour des techniques non chirurgicales, en particulier pour réduire la présence d'excès localisés de tissus adipeux.

L'adipocytolyse représente actuellement une de ces techniques non chirurgicales. Elle est utilisée comme traitement non chirurgical définitif des stéatoméries, mais également de la cellulite esthétique, agissant directement sur le tissu adipeux par rupture de la membrane cellulaire des adipocytes.

Cette technique repose sur l'effet de pression osmotique, dont l'unité physique de mesure est l'osmole (Osm), exprimée en mole par litre. Ainsi, lorsqu'une mole d'une substance est dissoute dans un litre d'eau, elle exerce une pression osmotique d'une osmole. L'osmolarité est la concentration en substance dissoute exerçant un pouvoir osmotique. Par exemples, l'osmolarité du plasma est considérée comme étant comprise entre 280 mOsm/L et 330 mOsm/L, celle du cytoplasme cellulaire est de 394 mOsm/L. Ainsi, les termes "hypotonie", "isotonie" et "hypertonie" sont utilisés ici pour désigner respectivement un compartiment dont l'osmolarité est inférieure, égale et supérieure, à l'osmolarité du plasma.

Plus précisément, l'effet lytique de cette technique d'adipocytolyse est obtenu par la combinaison d'effets physico-chimiques et biophysiques. Il débute tout d'abord par l'injection intra-graisseuse d'une solution fortement hypotonique (entre 60 mOsm/L et 90mOsm/L). Cette injection crée une différence de pression osmotique de part et d'autre de la membrane (semi-perméable) des adipocytes, ce qui provoque leur hyperhydratation, du fait d'un flux de liquide depuis le compartiment de plus faible osmolarité (le liquide interstitiel) vers le compartiment de plus forte osmolarité (le cytoplasme des adipocytes). Cela conduit à l'augmentation du volume des adipocytes, ainsi qu'à la fragilisation de leur membrane, voire à des lésions cellulaires. La destruction des adipocytes est ensuite complétée par un traitement biophysique léger, tel que l'application d'un champ ultrasonore transcutané. La lésion, puis la destruction des adipocytes provoque un relargage des graisses, ainsi que des triglycérides, acides gras libres et autres contenus cellulaires dans le plasma, qui seront dégradés par le foie pour être éliminés. Les débris cellulaires sont pris en charge par les monocytes et les macrophages.

Le brevet FR 2 876 907 A1 tente de remédier aux inconvénients que sont en particulier ceux de la répétition d'injections de solutions fortement hypotoniques, du recours à une lipo-aspiration complémentaire, et ce grâce à l'administration d'une solution iso- ou hypotonique et hyperkaliémique, préalablement au traitement esthétique par adipocytolyse susmentionné. Cela présente l'avantage de diminuer le volume des adipocytes, et ainsi de cibler un maximum de cellules à la fois au cours de l'étape d'injection intra-graisseuse de la solution fortement hypotonique. Ce brevet divulgue en outre une synergie d'action entre une solution isotonique hyperkaliémique, ou une solution hypotonique éventuellement hyperkaliémique avec certaines substances telles que le tiratricol (acide triiodothyroacétique) ou un composé amphotère tel que la phosphatidylcholine.

Il n'en demeure pas moins qu'un des problèmes majeurs de cette technique d'adipocytolyse reste l'optimisation de la composition des solutions fortement hypotoniques. En effet, l'injection de solutions hypotoniques, voire fortement hypotoniques peut faire augmenter le volume des adipocytes mais sans nécessairement conduire à leur éclatement, rendant ainsi le traitement par adipocytolyse inefficace.

C'est pourquoi, la présente invention se propose de résoudre le problème d'une lyse efficace des membranes des adipocytes lors d'un traitement de la stéatomérie, de la cellulite, ainsi que du lipome qui consiste en un traitement par adipocytolyse.

Les inventeurs ont développé des solutions salines aqueuses ayant une osmolarité comprise entre 50 mOsm/L et 170 mOsm/L, de préférence entre 50 mOsm/L et 100 mOsm/L qui comprennent en outre au moins un fragilisant membranaire.

Dans le cadre de la présente invention, on entend par fragilisant membranaire un composé qui est approprié pour exercer une action fragilisante sur la membrane de cellules, en particulier des adipocytes. Lesdits composés doivent être de puissants solubilisateurs de la bicouche de lipides dont sont constituées les membranes des cellules. Pour ce faire, ces composés s'insèrent dans la bicouche de lipides de manière à la déstabiliser par la création de pores et à la désorganiser; ce qui a pour conséquence la lyse des cellules. Les publications suivantes :
- "Current techniques for single-cell lysis", J.R. Soc. Interface (2008) 5, S131-S138, de BROWN et al.
- "Mixed micelles and other structures in the solubilization of bilayer lipid membranes by surfactants" Biochim Biophys Acta (2000); 1508: 146-63, de ALMGREN,
détaillent plus amplement le mode d'action de ces fragilisants membranaires décrit ci-dessus.

Le fragilisant membranaire peut être choisi parmi les détergents ioniques, les détergents non-ioniques, les détergents zwittérioniques, et des dérivés du glycérol.

A titre d'exemples, parmi les détergents non-ioniques, on peut notamment citer :
- les alkylglucosides tels que le n-octyl beta D glucopyranoside,
- les glucamides, tels que le N,N'-Bis(3-D-gluconamidopropyl)cholamide), connu sous la dénomination commerciale Big CHAP,
- les polyoxyéthylènes tels que les poloxamères, le 2,3-dihydroxypropyl octanoate (TWEEN® 20), le 2-[4-(2,4,4-trimethylpentan-2-yl)phenoxy]ethanol (TRITON® X 100), le 2-dodecoxyethanol (Brij® 35).

Parmi les détergents ioniques, on peut notamment citer les sels d'acides biliaires et leurs dérivés, ainsi que les sels d'alkyl ammonium, les dérivés d'acides gras des acides aminés, les carnitines, les dérivés glycérides d'acides aminés, les acyl lactilates, les esters d'acide tartrique mono-, diacetylé de mono- et diglycérides, les monoglycérides succinoylés, les esters d'acide citrique de mono- et de diglycérides, les sels d'alginate, l'alginate de propylène glycol, les lécithines et lécithines hydrogénées, les lysolecithines et les lysolecithines hydrogénées, les lysosphospholipides et leurs dérivés, les phospholipides et leurs dérivés, les sels d'alkylsulfates, les sels d'acides gras, le docusate de sodium.

Parmi les détergents zwittérioniques, on peut notamment citer le 3-[(3-Cholamidopropyl)dimethylammonio]-1-propanesulfonate, le N-tetradecyl-N,N-di methyl-3-ammonium-1-propanesulfonate, le lauryldimethylbetaine.

Les inventeurs ont trouvé que l'adjonction à des solutions salines aqueuses d'osmolarité comprise entre 50 mOsm/L et 170 mOsm/L de ces différents fragilisants membranaires a une action fragilisante sur la membrane des adipocytes, et plus particulièrement lorsque l'osmolarité est de 60 mOsm/L. Ainsi, ces solutions salines aqueuses permettent de favoriser la lyse des adipocytes lors de leur injection intra-graisseuse au cours d'un traitement de la cellulite, de la stéatomérie, ainsi que du lipome qui peut consister en un traitement par adipocytolyse.

De manière préférée, on utilise les sels d'acides biliaires et les poloxamères comme fragilisants membranaires ; les poloxamères étant tout particulièrement préférés.

Les inventeurs ont découvert l'effet synergique entre les sels d'acides biliaires et/ou les poloxamères dans des solutions d'osmolarité comprises entre 50 et 170 mOsm/L pour favoriser la lyse des adipocytes, et plus particulièrement lorsque l'osmolarité est de 60 mOsm/L.

Les sels d'acide biliaire constituent une large famille de molécules, ayant la structure d'un stéroïde (à savoir un hydrocarbure tétra-cyclique) comportant une chaîne latérale comprenant cinq à huit atomes de carbone et se terminant par une fonction carboxylate.

Dans le cadre de la présente invention, les sels d'acide biliaire peuvent être choisis parmi les sels suivants : le désoxycholate de sodium (DC), le tauro-désoxycholate de sodium (TDC), le lithocholate de sodium (LC) et le tauro-lithocholate de sodium (TLC), ou encore le sel d'acide cholique, le sel d'acide chénodésoxycholique, le sel d'acide 7-alpha-dehydroxylate chenodesoxycholique, le sel d'acide ursodésoxycholique, le sel d'acide dihydroxytaurique, le sel d'acide trihydroxytaurique, et les sels de leurs conjugués de glycine.

De manière préférée, le TDC ou le TLC sont choisis comme sel d'acide biliaire.

Les sels d'acide biliaire sont présents dans les solutions salines aqueuses selon l'invention à une concentration comprise entre 50µM et 3000µM, de préférence entre 50 µM et 1000 µM, encore plus préférentiellement entre 150µM et 750µM.

La structure des poloxamères (aussi connus sous le nom commercial de blocs de copolymère Pluronic® et commercialisés par la société BASF) ajoutés aux solutions salines aqueuses selon l'invention est décrite dans la publication de A. KABANOV, intitulée « Pluronic® block copolymers : novel functional molecules for gene therapy », publiée dans Advanced Drug Delivery Reviews 54 (2002) 223-233. Il s'agit de blocs d'oxyde d'éthylène (EO) et d'oxyde de propylène (PO) arrangés selon la structure tri-blocs suivante : EOₓ - PO_{y} - EOₓ.

Cet arrangement conduit à un copolymère amphiphile, dans lequel le nombre d'unités EO(x) hydrophiles et le nombre d'unités PO(y) hydrophobes peuvent être modifiés pour varier en taille, en hydrophilicité, et lipophilicité. Ces poloxamères, connus pour leur absence de toxicité, sont habituellement utilisés pour leurs propriétés tensio-actives.

Par la suite, on entendra par :
- « partie hydrophile du poloxamère » : l'ensemble des blocs EO dudit poloxamère,
- « partie hydrophobe du poloxamère » : l'ensemble des blocs PO dudit poloxamère.

La formule structurelle des poloxamères est la suivante (I) :

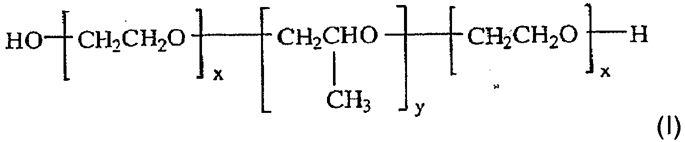

Formule dans laquelle x et y représentent des nombres entiers.

Dans la formule (I) ci-dessus :
- le bloc CH₂CH₂O est donc le bloc ci-avant dénommé EO,
- le groupe 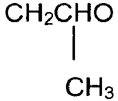 est donc le bloc ci-avant dénommé ci-avant PO.

Des exemples de poloxamères peuvent être les composés suivants, disponibles auprès de la société BASF:
- Pluronic® L61 EO₂- PO₃₀ - EO₂ MW = 1 950,
- Pluronic® P85 EO₂₆- PO₄₀- EO₂₆ MW = 4 600.

La nomenclature de ces poloxamères Pluronic® est détaillée dans l'appendice A de la publication de KABANOV précitée. Les lettres « F », « L », « P » indiquent respectivement un état solide, liquide ou pâteux du poloxamère. Les chiffres indiqués définissent les paramètres structuraux de ces polymères. Ainsi, le dernier chiffre indique approximativement la teneur en en poids de la partie hydrophile du poloxamère, exprimée en « dizaines ». Par exemple, si le dernier chiffre est 8, cela signifie que la partie hydrophile représente 80% du poids du poloxamère. Les autres chiffres restants (un ou deux) sont indicatifs de la masse molaire de la partie hydrophobe du poloxamère. Pour cela, il s'agit de multiplier le nombre correspondant par 300 afin d'obtenir la masse moléculaire approximative, exprimée en Da.

A titre d'exemple de nomenclature, le poloxamère F127 est à l'état solide, la masse de la partie hydrophobe de ce poloxamère est de 3600 Da (12 x 300), et la partie hydrophile représente 70% du poids du poloxamère.

D'après la publication de I.R. SCHMOLKA, intitulée « A review of block polymer surfactants », et publiée dans J. Am. Oil Chem. Soc. 54 (1977) 110-116, les poloxamères peuvent être synthétisés par polymérisation séquentielle de monomères PO et EO, en présence d'un catalyseur alcalin, tel que l'hydroxyde de sodium ou de potassium.

Dans le cadre de la présente invention, on peut utiliser au moins un poloxamère de formule structurelle (I) telle que définie ci-dessus comprenant une partie hydrophile et une partie hdrophobe, x et y étant choisis de manière à ce que la partie hydrophile représente 10 à 80% en poids du poloxamère, de préférence 30 à 50%, et à ce que la HLB (« hydrophilic/lipophilic balance ») soit inférieure à 35, de préférence inférieure à 20.Les valeurs de HLB sont déterminées par la procédure décrite dans la publication J. Am. Oil Chem. Soc. 41 (1964) :169, dont les auteurs sont BECHER, P. et BIRKMEIER R.L., qui met en oeuvre une chromatographie sur colonne dont le solvant est un mélange de n-hexane et d'éthanol.

Ainsi, dans le cadre de la présente invention, les poloxamères commercialisés par la société BASF que sont Pluronic® : L31, L35, F38, L42, L43, L44, L61 à L64, L72, P75, F77, L81, P75, P84, P85, F87, F88, L92, F98, L101, P103 à P105, F108, L121, L122, P123 peuvent être présents dans les solutions salines aqueuses selon l'invention, et dont les caractéristiques du poids en % de la partie hydrophile et la HLB sont présentées dans le tableau ci-après :

**Tableau 1 : Caractéristiques du % de la partie hydrophile et de la HLB de poloxamères.**

| Pluronic® | HLB | % partie hydrophile |
|---|---|---|
| L31 | 5 | 10 |
| L35 | 19 | 50 |
| F38 | 31 | 80 |
| F68 | 29 | 80 |
| L42 | 8 | 20 |
| L43 | 12 | 30 |
| L44 | 16 | 40 |
| L61 | 3 | 10 |
| L62 | 7 | 20 |
| L63 | 11 | 30 |
| L64 | 15 | 40 |
| L72 | 17 | 20 |
| P75 | 14 | 50 |
| P84 | 14 | 40 |
| P85 | 16 | 50 |
| F87 | 24 | 70 |
| F88 | 28 | 80 |
| L92 | 6 | 20 |
| F98 | 28 | 80 |
| L101 | 1 | 10 |
| P103 | 9 | 30 |
| P104 | 13 | 40 |
| P105 | 15 | 50 |
| F108 | 27 | 80 |
| L121 | 1 | 10 |
| L122 | 4 | 20 |
| P123 | 8 | 30 |

L'invention concerne une solution saline aqueuse telle que décrite ci-dessus, dans laquelle le poloxamère est choisi parmi les poloxamères de formule (I) qui ont :
- une partie hydrophile représentant 40% du poloxamère et la HLB est de 16,
- une partie hydrophile représentant 10% du poloxamère, et la HLB est de 3,
- une partie hydrophile représentant 20% du poloxamère, et la HLB est de 7,
- une partie hydrophile représente 30% du poloxamère, et la HLB est de 11,
- une partie hydrophile représentant 40% du poloxamère, et la HLB est de 15,
- une partie hydrophile représentant 50% du poloxamère, et la HLB est de 14,
- une partie hydrophile représentant 40% du poloxamère, et la HLB est de 14, et
- une partie hydrophile représentant 50% du poloxamère, et la HLB est de 16,
- une partie hydrophile représentant 30% en poids du poloxamère, et la HLB est de 8.

De manière appropriée, on utilise de préférence les poloxamères Pluronic® :
- L44, dont la partie hydrophile représente 40% en poids du poloxamère, et la HLB est de 16,
- L61, dont la partie hydrophile représente 10% en poids du poloxamère, et la HLB est de 3,
- L62, dont la partie hydrophile représente 20% en poids du poloxamère, et la HLB est de 7,
- L63, dont la partie hydrophile représente 30% en poids du poloxamère, et la HLB est de 11,
- L64, dont la partie hydrophile représente 40% en poids du poloxamère, et la HLB est de 15,
- P75, dont la partie hydrophile représente 50% en poids du poloxamère, et la HLB est de 14,
- P84 dont la partie hydrophile représente 40% en poids du poloxamère, et la HLB est de 14,
- P85, dont la partie hydrophile représente 50% en poids du poloxamère, et la HLB est de 16,
- P123, dont la partie hydrophile représente 30% en poids du poloxamère, et la HLB est de 8.

Selon la présente invention, les poloxamère Pluronic® P85, L61 et 64 sont des poloxamères préférés. Avantageusement, on utilise un poloxamère de formule (I) qui a une partie hydrophile représentant 30% en poids du poloxamère, et la HLB est de 8, et de préférence le Pluronic® P123.

Les poloxamères peuvent être présents dans les solutions salines aqueuses selon l'invention à une concentration comprise entre 0,001 % et 5% en poids/volume, de préférence entre 0,1% et 1%.

Un objet de la présente invention est une solution saline aqueuse ayant une osmolarité comprise entre 50 mOsm/L et 170 mOsm/L, de préférence entre 50 mOsm/L et 100 mOsm/L, qui comprend au moins un sel d'acide biliaire.

Un autre objet de la présente invention est une solution saline aqueuse ayant une osmolarité comprise entre 50 mOsm/L et 170 mOsm/L, de préférence entre 50 mOsm/L et 100 mOsm/L, qui comprend au moins un poloxamère.

Un autre objet de la présente invention est une solution saline aqueuse ayant une osmolarité comprise entre 50 mOsm/L et 170 mOsm/L, de préférence entre 50 mOsm/L et 100 mOsm/L, qui comprend au moins un poloxamère et au moins un sel d'acide biliaire.

Il est connu de WO 2005/063169 A2 et de WO 2005/041919 A2, des solutions aqueuses contenant au moins un phospholipide et/ou au moins un acide biliaire qui peuvent être utilisées dans la préparation de médicaments destinés au traitement de l'accumulation de tissus adipeux. Il est en outre précisé que ces solutions peuvent être diluées dans une solution saline. L'osmolarité des solutions aqueuses n'est nullement précisée dans ces documents, en particulier la nécessité que les solutions soient hypotoniques n'est nullement évoquée. Cependant, il est connu dans le domaine médical qu'une solution saline (par exemple une solution de NaCl à une concentration de 9g/L) peut consister en un liquide physiologique, à savoir un liquide présentant une osmolarité semblable aux principaux fluides corporels, tels que le sang, donc de l'ordre de 300 mOsm/L.

Dans le cadre de la présente invention, les solutions salines aqueuses sus-évoquées peuvent être des solutions contenant essentiellement les sels de NaCl, NaHCO₃, de MgCl₂, ayant des concentrations pouvant être respectivement comprises entre 10 et 25 mmol/L, 5 et 15 mmol/L, et 0,5 et 5 mmol/L.

En outre, les solutions salines aqueuses selon l'invention sont dépourvues de sels de potassium. En effet, la présence de potassium peut s'avérer douloureuse et dangereuse, en particulier chez les patients présentant une cardiopathie.

De plus, de manière préférée, la solution saline aqueuse selon l'invention contient du magnésium ; cet élément permettant de lutter contre un stress oxydant et inflammatoire qui peut se produire lors d'un traitement par ad ipocytolyse.

Dans le cadre de la présente invention, d'autres substances peuvent être présentes dans les solutions salines aqueuses selon l'invention ou ajoutées de manière extemporanée. Il peut s'agir d'adrénaline, d'un anesthésique tel que la lidocaïne.

L'adrénaline peut avoir une concentration comprise entre 0,5 et 2 mg/L. L'anesthésique tel que la lidocaïne peut avoir une concentration comprise entre 1 à 5 % en poids/volume.

De manière préférée, les solutions salines aqueuses selon l'invention comprennent de l'adrénaline à une concentration de 1 mg/L et de la lidocaïne à hauteur de 2% en poids/volume.

Le mélange de ces substances avec les acides biliaires et les poloxamères est tout à fait appréciable. En effet, une synergie est observée entre ces composés et les substances susmentionnées, ce qui permet de pouvoir diminuer les concentrations des composés que sont le sel d'acide biliaire et le poloxamère. On a ainsi pu diminuer la concentration en sel d'acide biliaire de l'ordre de 50 % pour obtenir le même effet de lyse des adipocytes.

La présente invention a aussi pour objet l'utilisation d'une solution saline aqueuse selon l'invention comme médicament.

La présente invention concerne aussi l'utilisation non thérapeutique d'une solution saline aqueuse selon l'invention pour réaliser la lyse des adipocytes.

La présente invention concerne aussi une composition cosmétique se caractérisant en ce qu'elle comprend une solution saline aqueuse selon l'invention.

L'invention concerne aussi l'utilisation d'une composition cosmétique selon l'invention pour le traitement non thérapeutique de la cellulite, de la stéatomérie, ainsi que du lipome.

Par ailleurs, l'invention concerne aussi l'utilisation d'une solution saline aqueuse selon l'invention pour l'obtention d'un médicament destiné au traitement de la cellulite, de la stéatomérie ainsi que du lipome.

La présente invention concerne aussi un dispositif médical pour le traitement de la cellulite, de la stéatomérie ainsi que du lipome.

La présente invention concerne aussi une méthode de traitement esthétique consistant en l'administration d'une solution saline aqueuse selon la présente invention, afin de pouvoir agir en particulier sur la cellulite, la stéatomérie, ainsi que sur un lipome. Ladite administration peut être réalisée par une méthode d'injection intra-graisseuse. L'administration de la solution saline aqueuse selon l'invention peut être réalisée dans toutes les couches de graisse décrites ci-avant, à savoir :
- la graisse hypodermique,
- la graisse parallèle, et
- la graisse stéatomérique.

De manière préférée, l'administration de la solution saline aqueuse selon l'invention est effectuée dans la couche de graisse stéatomérique qui est la plus profonde.

Cela est particulièrement avantageux, car cette couche de graisse profonde peut tout à fait être atteinte et donc être traitée grâce à l'administration de la solution saline aqueuse selon l'invention, alors qu'elle est très peu sensible aux traitements diététiques.

Ainsi, la méthode de traitement esthétique selon la présente invention est particulièrement adaptée aux couches de graisses, telles que la couche de graisse stéatomérique, pour lesquelles les méthodes de régime habituelles demeuraient inefficaces, et donc pour lesquelles on ne disposait pas actuellement de méthode valable permettant de détruire ces tissus adipeux profonds.

La méthode de traitement esthétique selon l'invention permet d'apporter une correction efficace et relativement durable de la silhouette.

La présente invention concerne une méthode de traitement esthétique consistant en l'injection intra-graisseuse d'une solution saline aqueuse selon l'invention, à savoir une solution saline aqueuse d'osmolarité pouvant être comprise entre 50 et 170 mOsm/L, de préférence comprise entre 50 et 100 mOsm/L, et de manière particulièrement préférée de 60 mOsm/L, et pouvant comprendre, outre au moins un sel d'acide biliaire et/ou au moins un poloxamère tels que décrits ci-dessus, au moins une des substances telles que l'adrénaline ou un anesthésique tel que la lidocaïne.

Dans un mode de réalisation de l'invention, l'administration peut être réalisée de la manière suivante :
- après anesthésie locale par de faibles injections épidermiques de la solution selon l'invention contenant de la lidocaïne (de l'ordre de 100µL),
- la solution selon l'invention est injectée en sous-cutané dans le tissu adipeux ciblé, et ce à différentes profondeurs d'injection, mais toujours par le même point d'injection.

Ainsi, jusqu'à environ un volume final d'un litre peut être administré par injection, et réparti de manière homogène dans les zones à traiter que peuvent être les cuisses, les hanches, l'abdomen ou encore les bras.

Plus précisément, après repérage préalable par des courbes concentriques des zones à traiter et des points d'injection, lesdites injections sont effectuées sur une période pouvant s'étaler de 20 à 30 minutes, par méthode manuelle ou éventuellement automatique.

La méthode de traitement esthétique non thérapeutique selon l'invention, pour être tout à fait efficace, peut être achevée par un traitement aux ultra-sons (non focalisés à basses fréquences, de préférence à 0,4 MHz) pendant une durée de 15 à 20 minutes, à l'aide d'un appareil spécialement adapté. Cela permet ainsi d'obtenir de meilleurs résultats quant au traitement esthétique selon l'invention.

Dans un autre mode de réalisation de l'invention, les substances telles que l'adrénaline ou un anesthésique tel que la lidocaïne ne sont pas présentes dans la solution saline aqueuse selon l'invention, mais peuvent être mélangées extemporanément à l'injection intra-graisseuse avec une solution saline aqueuse selon l'invention.

Les exemples ci-après permettront d'illustrer la présente invention sans pour autant en limiter sa portée.

### Description des figures :

La figure 1 est un graphe représentant le diamètre d'adipocytes incubés en présence de solutions salines aqueuses d'osmolarités : 300 mOsm/L, 150 mMOsm/L, 90 mOsm/L en fonction du temps.
La figure 2 est un graphe représentant l'évolution du nombre d'adipocytes en fonction du temps pour des adipocytes incubés dans les solutions salines aqueuses d'osmolarités de 90, 150 et 300 mOsm/L.
La figure 3 est un graphe représentant le diamètre des adipocytes incubés dans une solution d'osmolarité de 300 mOsm/L) et de 150 mOsm/L en présence de TDC, à une concentration de 500 µM en fonction du temps d'incubation.
La figure 4 est un graphe représentant le nombre d'adipocytes en fonction du temps pour des adipocytes incubés dans :
   - une solution saline aqueuse d'osmolarité de 300 mOsm/L,
   - une solution saline aqueuse d'osmolarité de 150 mOsm/L,
   - une solution saline aqueuse d'osmolarité de 150 mOsm/L, comprenant du TDC à une concentration de 500 µM.
La figure 5 est un graphe représentant le nombre d'adipocytes en fonction du temps pour des adipocytes incubés dans :
   - une solution saline aqueuse d'osmolarité de 300 mOsm/L,
   - une solution saline aqueuse d'osmolarité de 90 mOsm/L,
   - une solution saline aqueuse d'osmolarité de 90 mOsm/L) comprenant du TDC à une concentration de 500 µM.
La figure 6 est un graphe représentant le nombre d'adipocytes en fonction du temps pour des adipocytes incubés dans :
   - une solution saline aqueuse d'osmolarité de 300 mOsm/L,
   - une solution saline aqueuse d'osmolarité de 300 mOsm/L comprenant du TDC à une concentration de 500 µM,
   - une solution saline aqueuse d'osmolarité de 150 mOsm/L comprenant du TDC à une concentration de 500 µM,
   - une solution saline aqueuse d'osmolarité de 90 mOsm/L comprenant du TDC à une concentration de 500 µM.

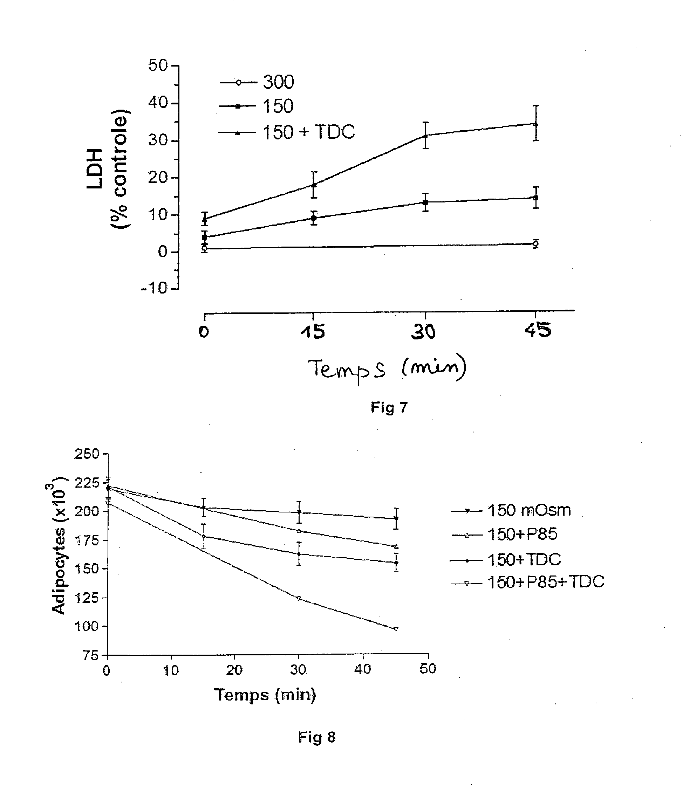

La figure 7 est un graphe représentant l'activité de la LDH en fonction du temps lors de l'incubation d'adipocytes dans respectivement :
   - une solution d'osmolarité de 300 mOsm/L,
   - une solution d'osmolarité de 150 mOsm/L,
   - une solution d'osmolarité de 150 mOsm/L comprenant du TDC, à une concentration de 500 µM,
La figure 8 est un graphe représentant le nombre d'adipocytes présents dans le milieu de culture en fonction du temps pour des adipocytes incubés dans :
   - une solution saline aqueuse d'osmolarité de 150 mOsm/L,
   - une solution saline aqueuse d'osmolarité de 150 mOsm/L comprenant du TDC à une concentration de 500 µM,
   - une solution saline aqueuse d'osmolarité de 150 mOsm/L comprenant du Pluronic® P85 à une concentration de 0,05%,
   - une solution saline aqueuse d'osmolarité de 150 mOsm/L comprenant du Pluronic® P85 à une concentration de 0,05% et du TDC à une concentration de 500 µM.

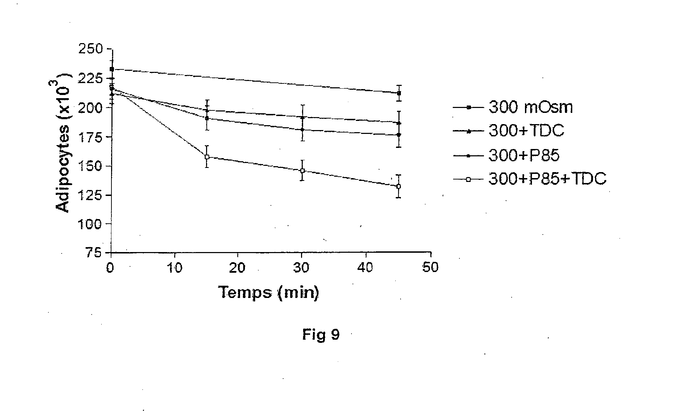

La figure 9 est un graphe représentant le nombre d'adipocytes présents dans le milieu de culture en fonction du temps pour des adipocytes incubés dans :
   - une solution saline aqueuse d'osmolarité de 300 mOsm/L,
   - une solution saline aqueuse d'osmolarité de 300 mOsm/L comprenant du TDC à une concentration de 500 µM,
   - une solution saline aqueuse d'osmolarité de 300 mOsm/L comprenant du Pluronic® P85 à une concentration de 0,05%,
   - une solution saline aqueuse d'osmolarité de 300 mOsm/L comprenant du Pluronic® P85 à une concentration de 0,05% et du TDC à une concentration de 500 µM.

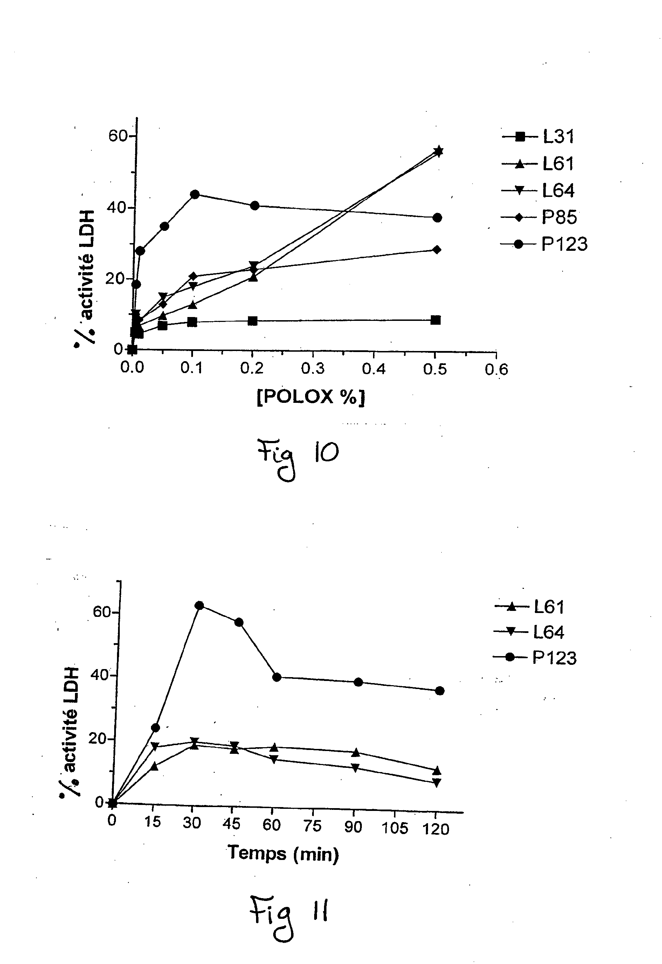

La figure 10 est un graphe représentant l'activité de la LDH libérée lors de la lyse complète des adipocytes en fonction de la concentration et de la nature de poloxamère présent dans une solution saline aqueuse d'osmolarité de 300 mOsm/L, et ce au bout d'une heure à 37°C.

La figure 11 est un graphe représentant l'activité de la LDH libérée lors de la lyse complète des adipocytes en fonction du temps et ce en fonction du choix du poloxamère présent dans une solution saline aqueuse d'osmolarité de 300 mOsm/L à une concentration de 0,1%.

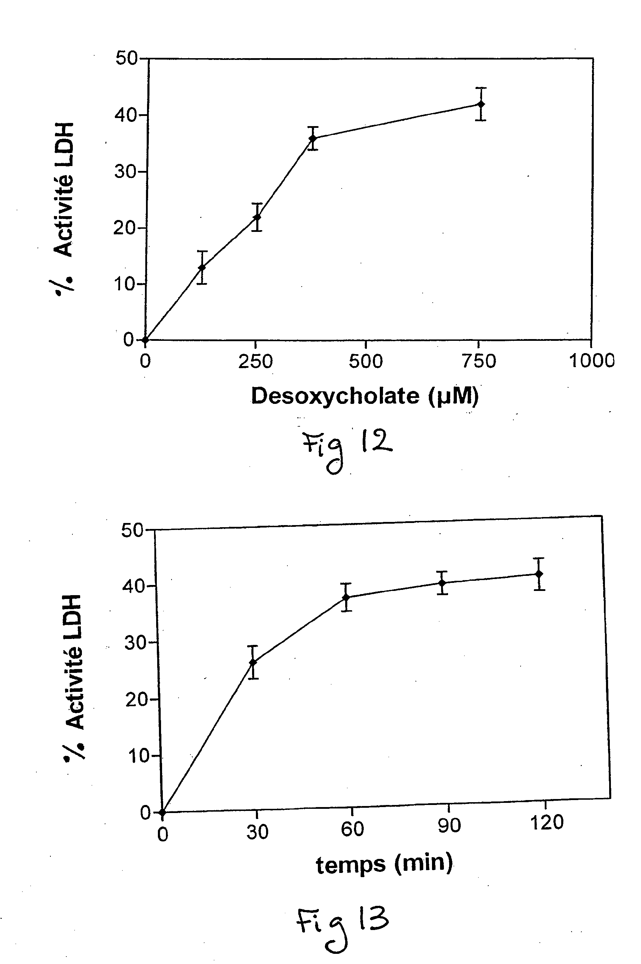

La figure 12 est un graphe représentant l'activité de la LDH libérée lors de la lyse complète des adipocytes au bout d'une heure en fonction de la concentration en sel d'acide biliaire DC présent dans une solution saline aqueuse d'osmolarité de 300 mOsm/L.

La figure 13 est un graphe représentant l'activité de la LDH libérée lors de la lyse complète des adipocytes en fonction du temps d'une solution saline aqueuse d'osmolarité de 300 mOsm/L qui comprend le DC comme sel d'acide biliaire et à une concentration de 750 µM.

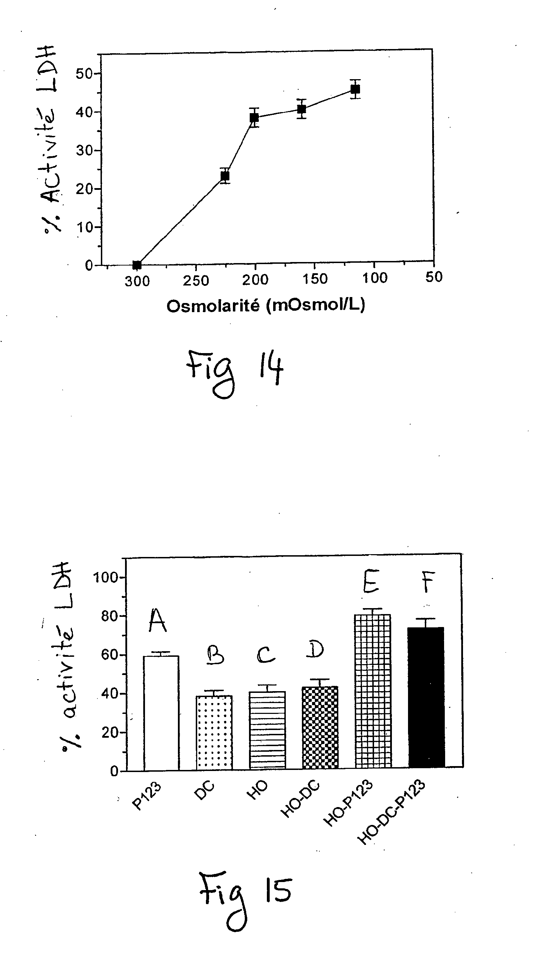

La figure 14 est un graphe représentant l'activité de la LDH au bout d'une heure en fonction de l'osmolarité d'une solution saline aqueuse.

La figure 15 représente un diagramme de l'activité de la LDH au bout d'une heure, pour :
- A: une solution saline aqueuse d'osmolarité de 300 mOsm/L comprenant 0,1% de P123,
- B : une solution saline aqueuse d'osmolarité de 300 mOsm/L comprenant 750 µM de DC,
- C : une solution saline aqueuse d'osmolarité de 160 mOsm/L,
- D : une solution saline aqueuse d'osmolarité de 160 mOsm/L comprenant 750 µM de DC,
- E: une solution saline aqueuse d'osmolarité de 160 mOsm/L comprenant 0,1% de P123,
- F : une solution saline aqueuse d'osmolarité de 160 mOsm/L comprenant 0,1% de P123 et 750 µM de DC.

### Exemples :

### A - Résultats expérimentaux obtenus à partir de tissus adipeux de souris :

Il est à noter que les exemples de cette partie A sont relatifs à des tests in-vitro utilisant des tissus adipeux de souris ; cela signifie que les cellules sont immergées dans le milieu de culture représentatif d'une solution saline aqueuse selon l'invention. Avec une osmolarité plus faible, la lyse des adipocytes lors d'un test in-vitro aurait été trop rapide et n'aurait donc pu être évaluée par décompte par exemple des adipocytes au cours du temps.

C'est pourquoi, l'osmolarité de ce milieu de culture décrit ci-après a dû être limitée à des valeurs supérieures ou égales à 90 mOsm/L et utilisé comme modèle représentatif de la situation in-situ qui est obtenue lors de la mise en oeuvre de l'invention sur des tissus adipeux in-situ.

### Préparation des adipocytes :

Des adipocytes ont été préparés à partir de tissus adipeux de souris de la manière suivante. Des tissus adipeux ont été prélevés en conditions aseptiques sur des animaux anesthésiés, puis finement découpés et soumis à un traitement dissociant enzymatique par de la collagénase type Il à 37°C. Le mélange d'incubation a ensuite été centrifugé pour éliminer les débris tissulaires. Les adipocytes ont été purifiés par filtration en conditions stériles sur un filtre en nylon de 160 µm. Après centrifugation douce, les adipocytes ont été lavés à partir d'une solution tampon de phosphate de pH 7,4 et, après numération, repris dans un milieu de culture cellulaire adapté de type Dubelco Modified Eagle's Medium ou DMEM, et enfin disposés en flacons de culture et incubateur stériles à 37°C adaptés aux expérimentations.

### Traitement des adipocytes préparés avec une solution selon l'invention :

Les adipocytes ainsi préparés ont été testés pendant une heure sur différentes solutions salines aqueuses comprenant des composés habituellement utilisés pour la culture cellulaire, tels que des acides aminés, des antibiotiques et d'osmolarité respectivement:
- normale, soit 300 mOsm/L,
- hypotonique, soit 150 mOsm/L,
- très hypotonique 90 mOsm/L.
et dans lesquelles avait été ajouté :
- soit un sel d'acide biliaire choisi parmi: le désoxycholate de sodium (DC), le lithocholate de sodium (LC), le tauro-désoxycholate de sodium (TDC), le tauro-lithocholate de sodium (TLC), à des concentrations variant de 100 µM à 750 µM,
- soit un poloxamère, à des concentrations comprises entre 0,02% et 1,5%,
- soit une combinaison d'acides biliaires et de poloxamères aux concentrations dans les intervalles précités.

Les conditions de mise en oeuvre des paramètres sus-évoqués ont été établies à partir de plans d'expérience.

La significativité des résultats obtenus a été étudiée à l'aide de méthodes statistiques pour la comparaison des moyennes, et au moyen de l'analyse de variance (ANOVA), suivies de tests adaptés (test de Dunett et de Bonferroni pour les comparaisons multiples).

### Examen des adipocytes traités :

### Détermination du nombre de cellules et de leur diamètre :

Les cellules graisseuses ont alors été examinées sous microscope inversé en conditions appropriées (grossissements identiques x200, compte sur 50 cellules) pour mesurer leur diamètre en fonction du temps d'exposition. Des photos ont été prises à intervalles de temps précis (à t = 0, 15, 30 et 45 min), afin de déterminer par des mesures automatiques le nombre de cellules et le diamètre des adipocytes. La mesure de la variation du diamètre des adipocytes a permis d'apprécier le gonflement des adipocytes, donc d'apprécier l'efficacité du traitement selon l'invention vis-à-vis du phénomène d'hyperhydratation.

La lyse cellulaire a été appréciée d'une part par la variation du nombre d'adipocytes, et d'autre part par le dosage d'une enzyme intracellulaire libérée dans le milieu de culture, la lactico-déshydrogénase (ci-après abrégée LDH), exprimée en pourcentage par rapport à des conditions pour lesquelles la lyse cellulaire est totale (obtenue en présence d'eau distillée) et par comparaison aux conditions de cultures cellulaires.

Ci-après, certains des résultats obtenus à partir des différentes combinaisons des paramètres susmentionnés sont présentés.

### Effet de l'hypotonicité seule:

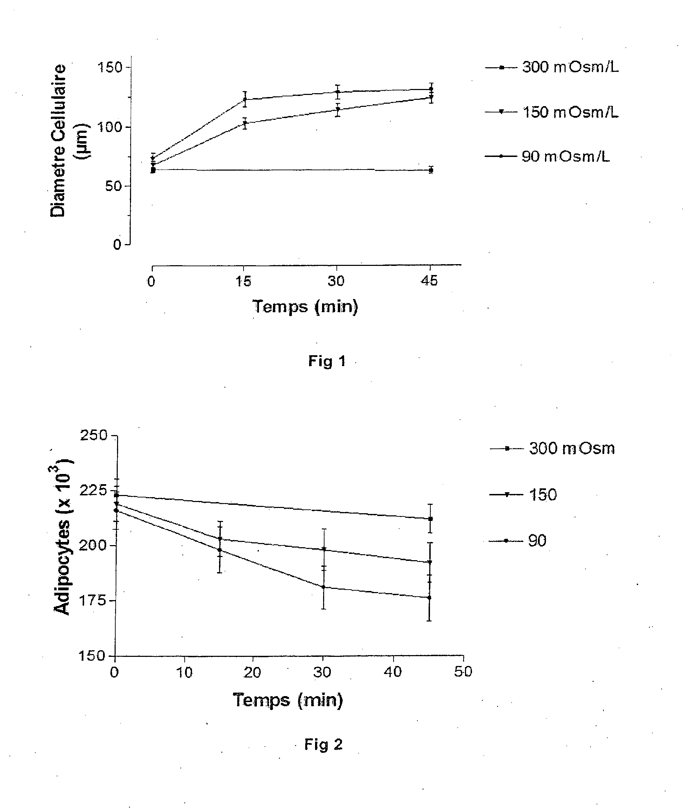

La figure 1 est un graphe représentant le diamètre (exprimé en µm) des adipocytes incubés en présence de solutions salines aqueuses d'osmolarités susmentionnées, à savoir isotonique (300 mOsm/L) ou hypotonique (150 mMOsm/L, et très hypotonique (90 mOsm/L), pour différents temps d'incubation (exprimés en minutes).

On constate une augmentation progressive du diamètre des adipocytes lors de leur incubation dans des solutions hypotoniques (150 et 90 mOsm/L) par rapport à celle lors de l'incubation dans une solution isotonique. L'augmentation du diamètre (comprise entre 50 et 85%) est relativement rapide et atteint un plateau vers 45 min. On relève aussi que ces augmentations du diamètre traduisant un gonflement cellulaire lors de l'incubation dans les solutions salines aqueuses sont plus rapides pour une solution très hypotonique 90 mMOsm/L que pour une solution hypotonique de 150 mOsm/L.

La figure 2 est un graphe représentant l'évolution du nombre d'adipocytes en fonction du temps pour des adipocytes incubés dans les solutions salines aqueuses susmentionnées du graphe de la figure 1, à savoir d'osmolarités de 90, 150 et 300 mOsm/L.

On constate de ce graphe de la figure 2 que plus l'osmolarité de la solution d'incubation des adipocytes est faible, plus le nombre d'adpicocytes diminue rapidement.

Ces premières mesures de l'effet de l'hypotonicité sur des adipocytes et rassemblées dans les graphes des figures 1 et 2 serviront de comparatif avec les mesures obtenues à partir de l'association d'une solution isotonique ou hypotonique avec un sel d'acide biliaire.

### Effet cumulé du sel de l'acide biliaire et de l'hypotonicité sur le diamètre des adipocytes:

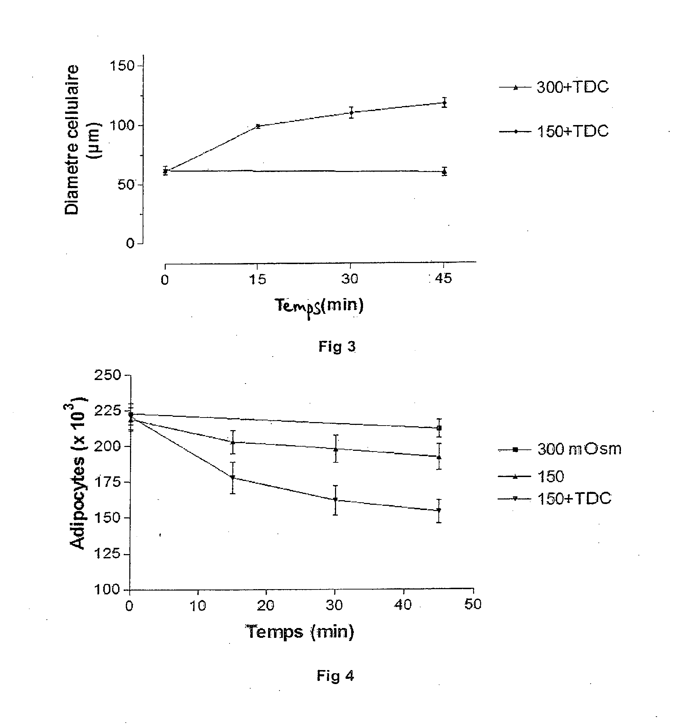

La figure 3 est un graphe représentant le diamètre des adipocytes incubés dans une solution isotonique (300 mOsm/L) et dans une solution hypotonique (150 mOsm/L) en présence de TDC, à une concentration de 500 µM, pour différents temps d'incubation (exprimés en minutes).

On constate que la présence de sel d'acide biliaire dans une solution isotonique ou hypotonique ne fait pas varier significativement le diamètre des adipocytes par rapport aux solutions isotonique ou hypotonique seules.

### Effet cumulé d'un sel d'acide biliaire et de l'hypotonicité sur le diamètre des adipocytes :

La figure 4 est un graphe représentant le nombre d'adipocytes en fonction du temps pour des adipocytes incubés dans :
- une solution saline aqueuse isotonique (300 mOsm/L),
- une solution saline aqueuse hypotonique (150 mOsm/L),
- une solution saline aqueuse hypotonique (150 mOsm/L), comprenant du TDC à une concentration de 500 µM.

On constate que le nombre d'adipocytes diminue plus rapidement et de manière plus importante, lorsque la solution saline aqueuse hypotonique comprend un sel d'acide biliaire par rapport à une même solution saline aqueuse mais dépourvue de ce sel d'acide biliaire. Cela témoigne de l'effet de la présence d'un sel d'acide biliaire dans une solution saline aqueuse selon l'invention sur la lyse des adipocytes.

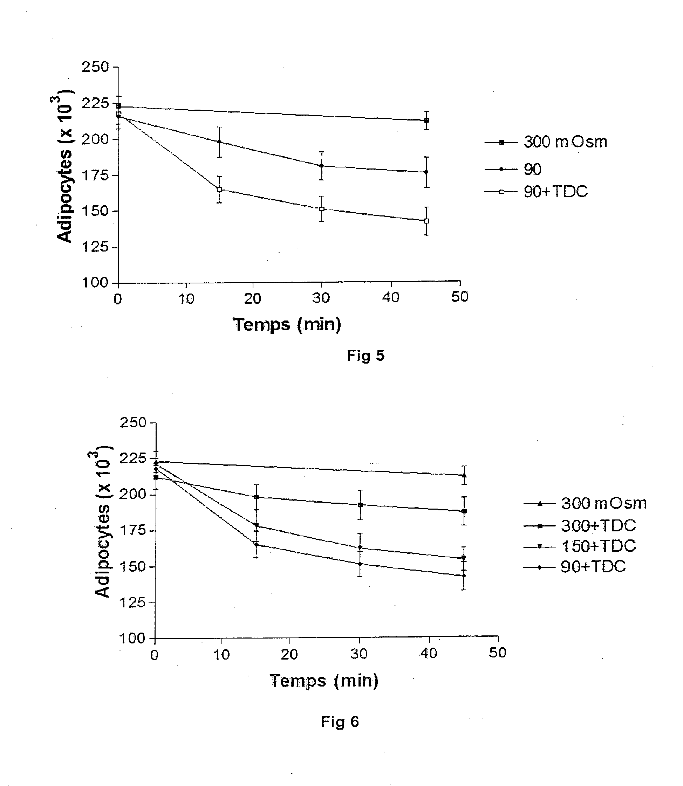

La figure 5 est un graphe représentant le nombre d'adipocytes en fonction du temps pour des adipocytes incubés dans :
- une solution saline aqueuse isotonique (300 mOsm/L),
- une solution saline aqueuse très hypotonique (90 mOsm/L),
- une solution saline aqueuse très hypotonique (90 mOsm/L) comprenant du TDC à une concentration de 500 µM.

On constate que le nombre d'adipocytes diminue plus rapidement et de manière plus importante lorsque la solution saline aqueuse très hypotonique comprend un sel d'acide biliaire par rapport à une même solution saline aqueuse dépourvu de sel d'acide biliaire. Cela témoigne de l'effet de la présence d'un sel d'acide biliaire dans une solution saline aqueuse selon l'invention sur la lyse des adipocytes.

La figure 6 est un graphe représentant le nombre d'adipocytes en fonction du temps pour des adipocytes incubés dans :
- une solution saline aqueuse isotonique (300 mOsm/L),
- une solution saline aqueuse isotonique (300 mOsm/L) comprenant du TDC à une concentration de 500 µM,
- une solution saline aqueuse hypotonique (150 mOsm/L) comprenant du TDC à une concentration de 500 µM,
- une solution saline aqueuse très hypotonique (90 mOsm/L) comprenant du TDC à une concentration de 500 µM.

On constate aussi que, lorsque la solution saline aqueuse isotonique (300 mOsm/L) contient un sel d'acide biliaire, le nombre d'adipocytes diminue à la différence d'une même solution isotonique, mais dépourvue de ce sel d'acide biliaire, pour laquelle le nombre d'adipocytes reste quasiment constant au cours du temps lors de l'incubation.

On constate aussi de ce graphe, que plus la solution saline aqueuse (et comprenant un sel d'acide biliaire) est hypotonique, plus le nombre d'adipocytes diminue rapidement et de manière plus importante.

### Effet cumulé d'un sel d'acide biliaire et de l'hypotonicité sur l'activité de la LDH :

La figure 7 est un graphe représentant l'activité de la LDH en fonction du temps lors de l'incubation d'adipocytes dans respectivement :
- une solution isotonique (300 mOsm/L),
- une solution hypotonique (150 mOsm/L),
- une solution hypotonique (150 mOsm/L) comprenant du TDC, à une concentration de 500 µM,

On constate que la solution saline aqueuse d'osmolarité de 150 mOsm/L induit une augmentation modérée de l'activité de la LDH dans le milieu, celle-ci est beaucoup plus augmentée lors de l'adjonction du TDC à une concentration de 500 µM à une même solution saline aqueuse d'osmolarité de 150 mOsm/L.

Effet cumulé de la combinaison d'un sel d'acide biliaire avec un poloxamère TDC et de l'hypotonicité sur le nombre d'adipocytes :

La figure 8 est un graphe représentant le nombre d'adipocytes présents dans le milieu de culture en fonction du temps pour des adipocytes incubés dans :
- une solution saline aqueuse hypotonique (150 mOsm/L),
- une solution saline aqueuse hypotonique (150 mOsm/L) comprenant du TDC à une concentration de 500 µM,
- une solution saline aqueuse hypotonique (150 mOsm/L) comprenant du Pluronic® P85 à une concentration de 0,05%,
- une solution saline aqueuse hypotonique (150 mOsm/L) comprenant du Pluronic® P85 à une concentration de 0,05% et du TDC à une concentration de 500 µM.

On constate que le nombre d'adipocytes diminue de plus en plus rapidement lorsque la solution saline aqueuse hypotonique contient respectivement :
- un poloxamère tel que le Pluronic® P85,
- un sel d'acide biliaire tel que le TDC,
- un mélange de poloxamère Pluronic® P85 et de sel d'acide biliaire TDC.

Le graphe de la figure 8 témoigne particulièrement de l'effet synergique de la combinaison du sel d'acide biliaire avec un poloxamère quant à la diminution du nombre d'adipocytes. En effet, entre 0 et 30 minutes, le nombre d'adipocytes diminue très fortement, et ce comparé aux autres essais susmentionnés et représentés dans ce même graphe.

Ainsi, cet exemple témoigne de l'effet synergétique du mélange d'un sel d'acide biliaire avec un poloxamère dans une solution saline aqueuse hypotonique (150 mOsm/L) quant à l'activité lytique de cette solution.

La figure 9 est un graphe représentant le nombre d'adipocytes présents dans le milieu de culture en fonction du temps pour des adipocytes incubés dans :
- une solution saline aqueuse isotonique (300 mOsm/L),
- une solution saline aqueuse isotonique (300 mOsm/L) comprenant du TDC à une concentration de 500 µM,
- une solution saline aqueuse isotonique (300 mOsm/L) comprenant du Pluronic® P85 à une concentration de 0,05%,
- une solution saline aqueuse isotonique (300 mOsm/L) comprenant du Pluronic® P85 à une concentration de 0,05% et du TDC à une concentration de 500 µM.

A partir de ce graphe de la figure 9, on constate aussi que le nombre d'adipocytes diminue de plus en plus rapidement lorsque la solution saline aqueuse hypotonique contient respectivement :
- un sel d'acide biliaire tel que le TDC,
- un poloxamère tel que le Pluronic® P85,
- un mélange de poloxamère Pluronic® P85 et de sel d'acide biliaire TDC.

Ainsi, le graphe de la figure 9 témoigne aussi de l'effet synergique de la combinaison du sel d'acide biliaire avec un poloxamère quant à la diminution du nombre d'adipocytes, et donc de l'effet lytique de ces solutions aqueuse.

Cependant, on constate que l'effet lytique est plus faible pour ces solutions salines aqueuses isotoniques par rapport à celui obtenu à partir de solutions hypotoniques (et faisant l'objet du graphe 8).

Par exemple, au bout de 45 minutes, le nombre d'adipocytes est de l'ordre de 100 x 10³ pour une solution saline aqueuse d'osmolarité de 150 mOsm/L comprenant un mélange de TDC et Pluronic® P85 et de l'ordre 140 x 10³ pour solution saline aqueuse d'osmolarité de 300 mOsm/L comprenant ce même mélange.

Ainsi, en comparant les résultats représentés sur les deux graphes des figures 8 et 9, on constate l'effet de l'osmolarité sur l'activité lytique des solutions salines aqueuses selon l'invention. Plus l'osmolarité de la solution saline aqueuse est faible, plus l'activité lytique est importante.

### B - Résultats expérimentaux obtenus à partir de tissus adipeux d'humain :

### Il - Résultats expérimentaux obtenus à partir de tissus adipeux d'humain :

Il est à noter que les exemples de cette 2^{ième} partie expérimentale sont relatifs à des tests in-vitro utilisant des tissus adipeux humains. L'hypo-osmolarité est relativement délicate à tester, car les cellules en culture possèdent une sensibilité particulière du fait de leur faible densité par comparaison à celle de tissus adipeux organisés. Dans les conditions expérimentales décrites ci-après, il n'a pas été possible de tester une hypo-osmolarité inférieure à 115 mOsmol/L du fait de la rapidité d'action de ce mode de lyse cellulaire (le décompte des adipocytes au cours du temps n'aurait pu être possible). De plus, la plupart des essais expérimentaux quant à la sélection du fragilisant membranaire de la solution saline aqueuse selon l'invention ont été réalisés avec une osmolarité de 300 mOsm/L (solution isotonique).

### Objectifs de cette 2^{ième} partie expérimentale :

Il s'agissait de déterminer les effets lytiques de solutions salines aqueuses selon l'invention sur des adipocytes humains en culture.
Pour ce faire, des adipocytes ont été incubés pendant des durées comprises entre 0 et 2 heures, dans des solutions salines aqueuses selon l'invention, et pour lesquelles les concentrations de poloxamères et de sel d'acide biliaire étaient croissantes et respectivement comprises entre 0,005 et 0,5% et 125 et 750 NM.

L'efficacité de la lyse adipocytaire a été appréciée par la mesure de l'activité d'une enzyme cytosolique : la lactico-déshydrogénase (LDH), libérée dans le milieu de culture et exprimée en pourcentage de la lyse totale.

### Aspects techniques et méthodologiques :

Les cellules étaient issues d'une lignée d'adipocytes humains (SW872) provenant d'un liposarcome déposé à la banque cellulaire américaine du National Institute of Health (NIH).
Les adipocytes ont été cultivés dans le milieu suivant : DMEM (Dubelco modified Eagle medium) à forte teneur en glucose (4,5 g/L) qui contenait du pyruvate, des antibiotiques (pénicilline/streptomycine), un supplément d'acides aminés et 25% de milieu de Ham-F12.
Il a été additionné à ce milieu de culture 10% de sérum de veau foetal pour l'entretien et la multiplication des adipocytes.
Il est à noter que les expérimentations ont ensuite été conduites en présence de 1% de sérum de veau foetal, afin de minimiser l'activité LDH basale susceptible d'être présente dans le sérum.
Les sels d'acides biliaires provenaient de la société SIGMA-ALDRICH.
Les poloxamères provenaient de la société BASF USA. Dans le cadre de cette 2^{ième} partie expérimentale, les poloxamères suivants ont été utilisés : L31, L61, L64, P85, P123.

Les sels d'acides biliaires et/ou les poloxamères ont été mis en solution dans le milieu de culture précité en conditions stériles. Des solutions salines aqueuses diluées adaptées ont été effectuées à partir de solutions mères à 2% pour les poloxamères et 3 mM pour le sel d'acide biliaire.

### Organisation générale du protocole :

Les adipocytes ont été mis en culture en conditions stériles (boîtes plastiques, incubateur à 5% C0₂, 37°C) dans des milieux spécifiques adaptés et utilisés pour des passages inférieurs à 10 de manière à réaliser des expérimentations sur des cellules primaires, c'est-à-dire proches des tissus adipeux d'origine. Les comptages cellulaires ont été effectués en cellules de Mallassez et la viabilité a été appréciée par l'exclusion du colorant bleu Trypan.

Après remise en suspension sous l'action de la trypsine, les adipocytes ont été incubés en plaques stériles 96 puits. Pour les expérimentations nécessitant d'importantes quantités de cellules, les incubations préalables ont été conduites en plaques stériles 24 puits, puis transférées en plaques 96 puits (fond rond) pour centrifugation, afin de séparer efficacement les cellules entières des cellules lysées ayant libéré leur contenu enzymatique retrouvé alors dans le surnageant. Une aliquote (100 µL) de ce dernier a ensuite été transférée dans une autre plaque 96 puits adaptées aux dosages (fond plat). Après addition du mélange de réactifs correspondants (kit BioChain #K6330400), l'activité LDH a alors été mesurée (après une incubation de 40 minutes avec agitation orbitale intermittente) dans un lecteur de plaques informatisé (MR5000 / Biolinx, Dynatech) à la longueur d'onde de 490 nm corrigée de l'absorbance à 630 nm. Les résultats sont exprimés en pourcentage de la lyse totale effectuée en présence de détergent (1% Triton X100).

Il est à noter qu'aucun problème évident de contamination, ni de viabilité cellulaire lors des cultures des adipocytes n'a été constaté; cette dernière est demeurée constamment supérieure à 97%. Il n'y a eu aucun problème de solubilisation des composés dans le milieu de culture pendant les expérimentations et y compris lors du stockage à 4°C.

### a) Activité de la LDH en fonction du poloxamère présent dans la solution saline aqueuse ainsi que de sa concentration :

La figure 10 est un graphe représentant l'activité de la LDH libérée lors de la lyse complète des adipocytes en fonction de la concentration (de 0 à 0,5%) et de la nature de poloxamère présent dans une solution saline aqueuse d'osmolarité de 300 mOsm/L, et ce au bout d'une heure à 37°C. Les poloxamères testés étaient le L31, le L61, le L64, le P85 et le P123.

On constate que le L31 a peu d'effet, l'activité de la LDH demeurant toujours inférieure à 10% et ce quelle que soit la concentration de ce poloxamère. On relève que l'activité de la LDH augmente proportionnellement avec l'augmentation de la concentration en L61 et L64. A une concentration de 0,5% de L61 ou L64, l'activité de la LDH s'élève à 56,5%. Pour les poloxamères P85 et P123, les effets lytiques atteignent des plateaux. On note cependant que le P123 a un effet important et ce même à de faibles concentrations, et cet effet se maintient aux fortes concentrations avec une activité de la LDH proche de 40%.

Ces résultats expérimentaux témoignent du fait que les poloxamères, de par leurs propriétés physico-chimiques, possèdent la propriété de lyser les adipocytes humains en culture. Les poloxamères P85, L64, L61 et P123 sont particulièrement efficaces ; le poloxamère L31 s'est avéré moins efficace dans ces conditions expérimentales.

Pour une concentration de 0,1%, on relève l'ordre d'efficacité décroissant suivant des poloxamères: P123>L64 L61>P85>L31.

Pour une concentration plus importante de 0,5%, l'ordre d'efficacité décroissant des poloxamères est sensiblement différent : L64=L61>P123>P85>L31.

De plus, les effets lytiques de certains poloxamères tels que les L61 et L64 s'avère dépendants de leur concentration, alors qu'un effet plateau est observé pour les poloxamères P85 et P123.

Ces différences d'action ne semblent pas être reliées à leur HLB puisque celles-ci sont différentes et que des poloxamères avec des HLB proches présentent des comportements très différents (par exemple, L64 et P85).

### b) Activité de la LDH en fonction du poloxamère présent dans la solution saline aqueuse ainsi que du temps :

La figure 11 est un graphe représentant l'activité de la LDH libérée lors de la lyse complète des adipocytes en fonction du temps (0 à 120 minutes), et ce en fonction du choix du poloxamère (L61, L64 ou P123) présent dans une solution saline aqueuse d'osmolarité de 300 mOsm/L à une concentration de 0,1%. La température du milieu ayant été maintenue à 37°C.

On constate que les effets de ces trois poloxamères sont rapides puisqu'une activité significative de la LDH est obtenue au bout de 15 minutes d'exposition. Les effets stagnent ou diminuent selon ces poloxamères au cours du temps. On relève que le P123 se maintient à une lyse proche de 40%.

### c) Activité de la LDH en fonction de la concentration du sel d'acide biliaire DC présent dans la solution saline aqueuse :

La figure 12 est un graphe représentant l'activité de la LDH libérée lors de la lyse complète des adipocytes au bout d'une heure en fonction de la concentration en sel d'acide biliaire DC (de 0 à 750 µM) présent dans une solution saline aqueuse d'osmolarité de 300 mOsm/L.

On constate une lyse maximale de 42±7% obtenue avec une incubation d'une heure en présence des adipocytes.

### d) Activité de la LDH en fonction du temps d'un solution saline aqueuse comprenant du sel d'acide biliaire DC à une concentration de 750 µM :

La figure 13 est un graphe représentant l'activité de la LDH libérée lors de la lyse complète des adipocytes en fonction du temps (0 à 120 minutes) d'une solution saline aqueuse d'osmolarité de 300 mOsm/L qui comprend le DC comme sel d'acide biliaire et à une concentration de 750 µM.

On constate qu'au bout de 30 minutes d'incubation, l'activité de la LDH s'élève à 26% et atteint une valeur de 37% au bout de 2 heures d'incubation.

### e) Activité de la LDH en fonction de l'osmolarité d'une solution saline aqueuse:

La figure 14 est un graphe représentant l'activité de la LDH au bout d'une heure en fonction de l'osmolarité d'une solution saline aqueuse (l'osmolarité variant entre 300 et 115 mOsm/L)

On constate une élévation de l'activité de la LDH proche de 25% pour une hypo-osmolarité abaissée à 225 mOsmol/L et atteignant 45±6% pour la plus faible osmolarité testée, soit 115 mOsmol/L.

### f) Activité de la LDH en fonction de la combinaison de différents paramètres (nature du fragilisant membranaire, osmolarité de la solution saline aqueuse) :

La figure 15 représente un diagramme de l'activité de la LDH au bout d'une heure, pour :
- A: une solution saline aqueuse d'osmolarité de 300 mOsm/L comprenant 0,1% de P123,
- B: une solution saline aqueuse d'osmolarité de 300 mOsm/L comprenant 750 µM de DC,
- C : une solution saline aqueuse d'osmolarité de 160 mOsm/L,
- D: une solution saline aqueuse d'osmolarité de 160 mOsm/L comprenant 750 µM de DC,
- E: une solution saline aqueuse d'osmolarité de 160 mOsm/L comprenant 0,1% de P123,
- F : une solution saline aqueuse d'osmolarité de 160 mOsm/L comprenant 0,1 % de P123 et 750 µM de DC.

On constate ainsi une activité lytique importante de l'ordre 59±5% du poloxamère P123 par comparaison à celle obtenue avec du sel d'acide biliaire DC à une concentration de 750 µM (38±5%). L'hypoosmolarité seule (160 mOsmol/L) entraîne une activité de la LDH de l'ordre de 40±8%. De plus, on relève l'effet synergique obtenu en combinant l'hypoosmolarité avec la présence de poloxamère P123 avec avec l'obtention d'une activité de la LDH s'élevant à 79±7%. On remarque par ailleurs que l'addition du DC à une solution saline aqueuse hypoosmolaire n'induit pas une lyse supplémentaire, puisque l'activité de la LDH est proche de 72±8%.

Les résultats expérimentaux décrits ci-dessous, certes obtenus dans des conditions différentes (à savoir in-vitro et donc pour ce faire d'osmolarité appropriée) de celles de la mise en oeuvre de la solution saline aqueuse selon l'invention, tendent à soutenir l'efficacité de cette solution lors de sa mise en oeuvre in-vivo.

## Revendications

1. Solution saline aqueuse d'osmolarité comprise entre 50 mOsm/L et 170 mOsm/L, de préférence entre 50 et 100 mOsm/L, **caractérisée en ce qu'**elle comprend en outre au moins un fragilisant membranaire, ledit fragilisant membranaire étant au moins un sel d'acide biliaire et/ou au moins un poloxamère de formule structurelle (I) : 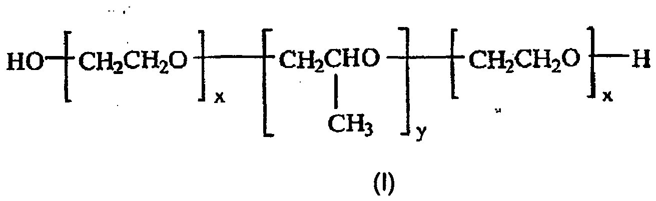 qui comprend une partie hydrophile et une partie hydrophobe, avec x et y étant des nombres entiers, et choisis de manière à ce que la partie hydrophile représente entre 10 et 80% du poids du poloxamère, de préférence entre 30 et 50% et que la HLB (hydrophilic/lipophilic balance) soit inférieure à 35, de préférence inférieure à 20.

2. Solution saline aqueuse selon la revendication 1, **caractérisée en ce que** la concentration en sel d'acide biliaire est comprise entre 50µM et 3000µM, de préférence entre 50 et 1000 µM, encore plus préférentiellement entre 150 et 750 µM et/ou la concentration en poloxamère est comprise entre 0,001% et 5% en poids/volume, de préférence entre 0,1% et 1% en poids/volume.

3. Solution saline aqueuse selon l'une quelconque des revendications 1 à 2, **caractérisée en ce que** le au moins un sel d'acide biliaire est choisi parmi le désoxycholate de sodium (DC), le tauro-désoxycholate de sodium (TDC), le lithocholate de sodium (LC) et le tauro-lithocholate de sodium (TLC).

4. Solution saline aqueuse selon l'une quelconque des revendications 1 à 3, **caractérisée en ce que** le au moins un poloxamère est choisi parmi les poloxamères de formule (I) qui ont :
- une partie hydrophile représentant 40% du poloxamère et la HLB est de 16,
- une partie hydrophile représentant 10% du poloxamère, et la HLB est de 3,
- une partie hydrophile représentant 20% du poloxamère, et la HLB est de 7,
- une partie hydrophile représente 30% du poloxamère, et la HLB est de 11,
- une partie hydrophile représentant 40% du poloxamère, et la HLB est de 15,
- une partie hydrophile représentant 50% du poloxamère, et la HLB est de 14,
- une partie hydrophile représentant 40% du poloxamère, et la HLB est de 14,
- une partie hydrophile représentant 50% du poloxamère, et la HLB est de 16,
- une partie hydrophile représentant 30% en poids du poloxamère, et la HLB est de 8.

5. Solution saline aqueuse selon l'une quelconque des revendications 1 à 4, **caractérisée en ce qu'**elle comprend au moins un sel choisi parmi NaCl à une concentration comprise entre 10 et 25 mmol/L, NaHCO₃ à une concentration comprise entre 5 et 15 mmol/L, MgCl₂ à une concentration comprise entre 0,5 et 5 mmol/L.

6. Solution saline aqueuse selon l'une quelconque des revendications 1 à 5, **caractérisée en ce qu'**elle comprend en outre au moins une substance choisie parmi l'adrénaline, un anesthésique tel que la lidocaïne.

7. Utilisation d'une solution saline aqueuse d'osmolarité comprise entre 50 mOsm/L et 170 mOsm/L, de préférence entre 50 et 100 mOsm/L, ladite solution saline aqueuse comprenant en outre au moins un fragilisant membranaire, pour l'obtention d'un médicament destiné au traitement de la stéatomérie ainsi que du lipome.

8. Utilisation selon la revendication 7, **caractérisée en ce que** le au moins un fragilisant membranaire est choisi parmi les détergents ioniques, les détergents non-ioniques, les détergents zwittérioniques et les dérivés du glycérol.

9. Utilisation selon la revendication 8, **caractérisée en ce que** le au moins un fragilisant membranaire est choisi parmi les alkylglucosides, les glucamides, les polyoxyethylènes, les sels d'acides biliaires.

10. Utilisation non-thérapeutique d'une solution saline aqueuse selon l'une quelconque des revendications 1 à 6 pour le traitement de la cellulite.

## Patentansprüche

1. Wässrige Salzlösung mit einer Osmolarität zwischen 50 mOsm/L und 170 mOsm/L, vorzugsweise zwischen 50 und 100 mOsm/L, **dadurch gekennzeichnet, dass** sie außerdem mindestens ein Membran-Versprödungsmittel umfasst, wobei das Membran-Versprödungsmittel mindestens ein Gallensäuresalz und/oder mindestens ein Poloxamer mit der folgenden strukturellen Formel (I) ist: 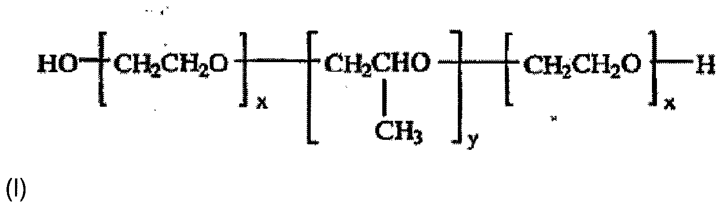 das einen hydrophilen Teil und einen hydrophoben Teil umfasst, wobei x und y ganze Zahlen sind, und derart ausgewählt, dass der hydrophile Teil zwischen 10 und 80 % des Gewichts des Poloxamers darstellt, vorzugsweise zwischen 30 und 50 %, und dass der HLB-Wert (hydrophilic/lipophilic balance) geringer als 35, vorzugsweise geringer als 20 ist.

2. Wässrige Salzlösung nach Anspruch 1, **dadurch gekennzeichnet, dass** die Konzentration von Gallensäuresalz zwischen 50 µM und 3000 µM, vorzugsweise zwischen 50 und 1000 µM, noch bevorzugter zwischen 150 und 750 µM liegt, und/oder die Konzentration von Poloxamer zwischen 0,001 und 5 Gew/Vol% des liegt, vorzugsweise zwischen 0,1 und 1 Gew/Vol%.

3. Wässrige Salzlösung nach einem der Ansprüche 1 bis 2, **dadurch gekennzeichnet, dass** das mindestens eine Gallensäuresalz ausgewählt ist aus Natriumdesoxycholat (DC), Natrium-Taurodesoxycholat (TDC), Natrium-Lithocholat (LC) und Natrium-Taurolithocholat (TLC).

4. Wässrige Salzlösung nach einem der Ansprüche 1 bis 3, **dadurch gekennzeichnet, dass** das mindestens eine Poloxamer ausgewählt ist aus den Poloxameren mit der Formel (I), die Folgendes aufweisen:
- einen hydrophilen Teil, der 40 % des Poloxamers aufweist, und wobei der HLB-Wert gleich 16 ist,
- einen hydrophilen Teil, der 10 % des Poloxamers aufweist, und wobei der HLB-Wert gleich 3 ist,
- einen hydrophilen Teil, der 20 % des Poloxamers aufweist, und wobei der HLB-Wert gleich 7 ist,
- einen hydrophilen Teil, der 30 % des Poloxamers aufweist, und wobei der HLB-Wert gleich 11 ist,
- einen hydrophilen Teil, der 40 % des Poloxamers aufweist, und wobei der HLB-Wert gleich 15 ist,
einen hydrophilen Teil, der 50 % des Poloxamers aufweist, und wobei der HLB-Wert gleich 14 ist,
- einen hydrophilen Teil, der 40 % des Poloxamers aufweist, und wobei der HLB-Wert gleich 14 ist,
- einen hydrophilen Teil, der 50 % des Poloxamers aufweist, und wobei der HLB-Wert gleich 16 ist,
- einen hydrophilen Teil, der 30 Gew% des Poloxamers aufweist, und wobei der HLB-Wert gleich 8 ist.

5. Wässrige Salzlösung nach einem der Ansprüche 1 bis 4, **dadurch gekennzeichnet, dass** sie mindestens ein Salz umfasst, ausgewählt aus NaCl mit einer Konzentration, die zwischen 10 und 25 mmol/L liegt, NaHCO₃ mit einer Konzentration, die zwischen 5 und 15 mmol/L liegt, MgCl₂ mit einer Konzentration, die zwischen 0,5 und 5 mmol/L liegt.

6. Wässrige Salzlösung nach einem der Ansprüche 1 bis 5, **dadurch gekennzeichnet, dass** sie außerdem mindestens eine Substanz umfasst, ausgewählt aus Adrenalin, einem Anästhetikum wie z.B. Lidocain.

7. Verwendung einer wässrigen Salzlösung mit einer Osmolarität zwischen 50 mOsm/L und 170 mOsm/L, vorzugsweise zwischen 50 und 100 mOsm/L, wobei die wässrige Salzlösung außerdem mindestens ein Membran-Versprödungsmittel umfasst, um ein Arzneimittel zu erhalten, das für die Behandlung der Steatomerie sowie des Lipoms ausgelegt ist.

8. Verwendung nach Anspruch 7, **dadurch gekennzeichnet, dass** das mindestens eine Membran-Versprödungsmittel ausgewählt ist aus den ionischen Tensiden, den nicht ionischen Tensiden, den zwitterionischen Tensiden und den Glycerolderivaten.

9. Verwendung nach Anspruch 8, **dadurch gekennzeichnet, dass** das mindestens eine Membran-Versprödungsmittel ausgewählt ist aus den Alkylglucosiden, den Glucamiden, den Polyoxyethylenen, den Gallensäuresalzen.

10. Nicht-therapeutische Verwendung einer wässrigen Salzlösung nach einem der Ansprüche 1 bis 6 zur Behandlung der Cellulitis.

## Claims

1. An aqueous saline solution with osmolarity comprised between 50 mOsm/L and 170 mOsm/L, preferably between 50 and 100 mOsm/L, **characterized in that** it further comprises at least one membrane-weakening agent, said membrane-weakening agent being at least one biliary acid salt and/or at least one poloxamer of structural formula (I): 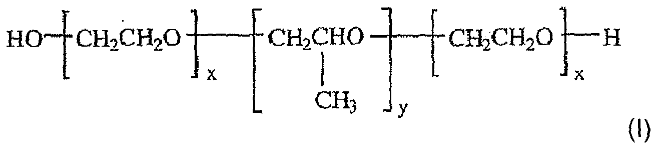 which comprises a hydrophilic portion and a hydrophobic portion, with x and y being integers, and selected so that the hydrophilic portion represents between 10 and 80% by weight of the poloxamer, preferably between 30 and 50% and the HLB (hydrophilic/lipophilic balance) is less than 35, preferably less than 20.

2. The aqueous saline solution according to claim 1, **characterized in that** the concentration of biliary acid salt is comprised between 50 µm and 3,000 µM, preferably between 50 and 1,000 µM, still more preferentially between 150 and 750 µM and/or the concentration of poloxamer is comprised between 0.001 % and 5% by weight/volume, preferably between 0.1 % and 1% by weight/volume.

3. The aqueous saline solution according to any of claims 1 to 2, **characterized in that** said at least biliary acid salt is selected from sodium deoxycholate (DC), sodium deoxycholate (TDC), sodium lithocholate (LC) and sodium tauro-lithocholate (TLC).

4. The aqueous saline solution according to any of claims 1 to 3, **characterized in that** said at least one poloxamer is selected from poloxamers of formula (I) which have:
- a hydrophilic portion representing 40% of poloxamer and the HLB is 16,
- a hydrophilic portion representing 10% of the poloxamer and the HLB is 3,
- a hydrophilic portion representing 20% of the poloxamer and the HLB is 7,
- a hydrophilic portion representing 30% of the poloxamer and the HLB is 11,
- a hydrophilic portion representing 40% of the poloxamer, and the HLB is 15,
- a hydrophilic portion representing 50% of the poloxamer and the HLB is 14,
- a hydrophilic portion representing 40% of the poloxamer and the HLB is 14,
- a hydrophilic portion representing 50% of the poloxamer and the HLB is 16,
- a hydrophilic portion representing 30% by weight of the poloxamer, and the HLB is 8.

5. The aqueous saline solution according to any of claims 1 to 4, **characterized in that** it comprises at least one salt selected from NaCl at a concentration comprised between 10 and 25 mOsm/L, NaHCO₃ at a concentration comprised between 5 and 15 mOsm/L, MgCl₂ at a concentration comprised between 0.5 and 5 mOsm/L.

6. The aqueous saline solution according to any of claims 1 to 5, **characterized in that** it further comprises at least one substance selected from adrenalin, an anaesthetic such as lidocaine.

7. Use of an aqueous saline solution with osmolarity comprised between 50 mOsm/L and 170 mOsm/L, preferably between 50 and 100 mOsm/L, said aqueous saline solution further comprising at least membrane-weakening agent, for obtaining a medicine intended for treating steatomery as well as lipoma.

8. Use according to claim 7, **characterized in that** said at least one weakening agent is selected from ionic detergents, non-ionic detergents, zwitterionic detergents and derivatives of glycerol.

9. Use according to claim 8, **characterized in that** said at least one membrane-weakening agent is selected from alkyl glucosides, glucamides, polyoxiethylene, biliary acid salts.

10. Non-therapeutic use of an aqueous saline solution according to any of claims 1 to 6 for treating cellulitis.
